# EUROPÄISCHE PATENTSCHRIFT

(11) **EP 1 838 259 B1**
(45) Veröffentlichungstag und Bekanntmachung des Hinweises auf die Patenterteilung: **31.12.2008**
(21) Anmeldenummer: 05817047.3
(22) Anmeldetag: 23.11.2005
(51) Int. Cl.: A61F 5/56

(54) **STUFENLOS VERSTELLBARE UNTERKIEFERPROTRUSIONSSCHIENE ZUR BEHANDLUNG VON SCHNARCHEN UND OBSTRUKTIVER SCHLAFAPNOE**
CONTINUOUSLY ADJUSTABLE MANDIBULAR PROTRUSION ORTHESIS FOR TREATING SNORING AND OBSTRUCTIVE SLEEP APNEA
ORTHESE D'AVANCEE MANDIBULAIRE A REGLAGE CONTINU POUR LE TRAITEMENT DU RONFLEMENT ET DE L'APNEE OBSTRUCTIVE DU SOMMEIL

(30) Priorität: 01.12.2004 DE 102004058081; 24.09.2005 DE 202005015106 U
(43) Veröffentlichungstag der Anmeldung: 03.10.2007
(73) Patentinhaber: Toussaint, Winfried, Dr., 64625 Bensheim (DE)
(72) Erfinder: Toussaint, Winfried, Dr., 64625 Bensheim (DE)
(74) Vertreter: Kompter, Hans-Michael
(86) Internationale Anmeldenummer: PCT/DE2005/002095
(87) Internationale Veröffentlichungsnummer: WO 2006/058514

(56) Entgegenhaltungen:
- EP-A- 0 359 135
- EP-A- 0 801 937
- US-A- 5 267 862
- US-A- 5 829 441
- US-A1- 2002 000 230
- US-A1- 2003 217 753
- US-B1- 6 305 376

## Beschreibung

### HINTERGRUND DER ERFINDUNG

### 1. TECHNISCHES GEBIET

Die Erfindung betrifft eine stufenlos verstellbare, zweiteilige Unterkieferprotrusionsschiene zur Behandlung von Schnarchen und/oder obstruktiver Schlafapnoe.

### 2. STAND DER TECHNIK

Schnarchen kann ein Symptom für das obstruktive Schlafapnoesyndrom sein, gekennzeichnet durch wiederholte und zahlreich auftretende nächtliche Atemstillstände, die schwerwiegende gesundheitliche Komplikationen, wie z.B. Bluthochdruck, Herz-Kreislauferkrankungen, Gehirnschlag u.a. nach sich ziehen können. Durch das US Patent US 5,462,066 sowie die europäische Patentanmeldung EP 1 203 570 sind derartige zahnspangenartige Aufbissschienen zur Verhinderung des Schnarchens bekannt geworden, welche dazu dienen, den Unterkiefer geringfügig nach vorne zu verschieben, weil in dieser Stellung des Unterkiefers die Atemwege weiter geöffnet werden, so dass der Patient freier atmen kann, ohne zu schnarchen.

Die bekannten Aufbissschienen in Form eines einteiligen, zahnspangenartigen Mundstücks bestehen aus thermoplastischen Materialien mit zwei Bissrillen, welche bei Erwärmung formbar werden. Der Patient nimmt das erwärmte, noch nicht angepasste Mundstück in den Mund, um dann in den formbaren Kunststoff die Zähne des Unter- sowie des Oberkiefers in die entsprechende untere sowie obere Bissrille hineinzudrücken und durch Aufbeissen auf die Bissplatten der Bissrillen anzupassen. Dabei kühlt der Kunststoff ab und gewinnt seine feste Elastizität zurück, wonach nunmehr das Mundstück an den Patienten angepasst ist. Beim Anpassungsvorgang muss darauf geachtet werden, den Unterkiefer etwas nach vorne zu verschieben, um dauerhaft einen Vorschub (Protrusion) einzustellen. Die bekannten Aufbissschienen besitzen den Nachteil, dass eine einmal eingestellte Protrusion nur schwierig sich ändernden Bedürfnissen des Patienten angepasst werden kann, so dass sich mit der Zeit der anfänglich erzielte Effekt verschlechtert.

Weiterhin schlägt das US Patent US 5,868,138 eine dentale Vorrichtung zum Behandeln von Schnarchen und obstruktivem Schlafatemstillstand vor, welches ein Oberkieferteil, ein Unterkieferteil und ein Verbindungsmittel aufweist, wobei das Verbindungsmittel das untere Teil relativ zum oberen Teil in einer vorderen vorstehenden Position einstellbar hält.

Dazu müssen individuell im Dentallabor nach Abdrucknahme Ober- und Unterkieferschienen angefertigt werden.

Als in der Regel aus dem Werkstoff Titan bestehendes Verbindungsmittel wird ein in verschiedenen Positionen des Unterteils stufenweise koppelbarer, senkrecht zu der Grundfläche des Oberteiles fest angebrachter Stift vorgeschlagen. In der europäischen Patentanmeldung EP 0 801 937 A wird eine zweiteilige Unterkieferprotrusionsschiene vorgeschlagen, die eine Führungseinrichtung zur Aufnahme eines Schraubenkopfes aufweist, wobei diese Führungseinrichtung jedoch keine relative laterale Bewegung des Oberkiefers bezüglich des Unterkiefers zulässt.

Der vorliegenden Erfindung lag die Aufgabe zu Grunde, Schnarchern und Schlafapnoikern im Vergleich dazu eine in der Wirksamkeit vergleichbare Unterkieferprotrusionsschiene, die eine lange Lebensdauer aufweist und leicht zu handhaben ist, zur Verfügung zu stellen. Zudem sollte die zu entwickelnde Schiene problemlos von jedem Arzt - nicht nur von Zahnärzten- oder sogar vom Patienten selbst angepasst werden können, was eine erheblich Vereinfachung in der Handhabung erforderlich macht. Des weiteren sollte der Unterkiefervorschub der erfindungsgemäßen Unterkieferprotrusionsschiene stufenlos an die Bedürfnisse des Patienten angepasst werden können.

Diese Aufgabe wurde erfindungsgemäß gelöst durch die Bereitstellung einer universellen zweiteiligen Unterkieferprotrusionsschiene umfassend ein Unter- (2) und ein Oberteil (3), welche jeweils zahnspangenartig dem menschlichen Ober- und Unterkiefer nachgeformt sind, wobei das Oberteil mit dem Unterteil über ein waagrecht zur Grundfläche der beiden Teile angeordnetes Verbindungsmittel so miteinander verbunden ist, dass das Unterteil in der Längsrichtung nach vorne stufenlos verstellbar ist.

### KURZE ZUSAMMENFASSUNG DER ERFINDUNG

Gegenstand der Erfindung ist somit eine universell einsetzbare zweiteilige Unterkieferprotrusionsschiene gemäß Anspruch 1.

In einer ersten, universellen Ausführungsform enthalten die formgebenden und biegesteifen Schale (2a, 3a)ein thermoplastisches Füllmaterial (4, 5), welches zahnspangenartig dem menschlichen Ober- und Unterkiefer nachformbar ist.

Diese Ausführungsform vermeidet die zeit- und kostenaufwendige individuelle Fertigung im Dentallabor nach vorheriger Zahnabdrucknahme durch einen Zahnarzt oder Kieferorthopäden. Zudem kann diese universelle Schiene problemlos von jedem Arzt - nicht nur von Zahnärzten- oder sogar vom Patienten selbst angepasst werden können, was eine erheblich Vereinfachung in der Handhabung erforderlich macht.

In einer weiteren individuellen Ausführungsform werden die formgebenden und biegesteifen Schalen ( 2a, 3a) aus handelsüblichen thermoformen Tiefziehplatten (z.B. Erkoloc Pro Tiefziehplatten der Fa. Erkodent GmbH, Duran Tiefziehplatten der Fa. Scheu Dental GmbH u.v.a) im so genannten Druckformverfahren nach Zahnabdrucknahme im Dentallabor individuell, abgestimmt auf die Zahn- und Kieferverhältnisse des Patienten, passgenau hergestellt. Bei dieser individuellen Ausführungsform wird das separate Verbindungselement (50), bestehend aus der Führungsschiene und der Schraubenführung, im Dentallabor an Ober- (Führungsschiene) und Unterschiene (Schraubenführung) befestigt.

Die erfindungsgemäßen Unterkieferprotrusionsschiene kann durch ein Verfahren umfassend die folgenden Schritte hergestellt werden:
(a) Formen der formgebenden und biegesteifen Schalen (2a, 3a) mit jeweils auf den Außenseiten der Bodenflächen vorgeformten Befestigungselementen im Spritzgußverfahren;
(b) Füllen der Schalen mit einem thermoplastisches Füllmaterial (4, 5) vorzugsweise per Spritzgussverfahren insbesondere bei Massenfertigung;
(c) Verbinden der vorgeformten Befestigungselemente.

Weiterhin kann eine erfindungsgemäße, individuell angepasste Unterkieferprotrusionsschiene durch ein Verfahren umfassend die folgenden Schritte hergestellt werden:
(i) Anfertigen einer Dentalschiene auf Grund eines Zahnabdruckes des Patienten;
(ii) Befestigen einer nach vorne geöffneten Führungsschiene auf der Unterseite der Oberkieferschiene;
(iii) Befestigen einer Schraubenführung auf der Unterseite der Unterkieferschiene
(iv) Verbinden der Schraubenführung mit der Führungsschiene mittels einer Schraube, deren Schraubenkopf von der Führungsschiene aufgenommen wird.

Ein weiterer Gegenstand der Erfindung ist ein gebrauchsfertiges Set zur Herstellung einer erfindungsgemäßen Unterkieferprotrusionsschiene zur Verhinderung von Schnarchen und/oder (obstruktiver) Schlafapnoe gemäß Auspcuch 8.

Eine vorteilhafte Ausgestaltung ist gebrauchsfertiges Set zur Herstellung einer erfindungsgemäßen Unterkieferprotrusionsschiene zur Verhinderung von Schnarchen und/oder (obstruktiver) Schlafapnoe bestehend aus
(A) einer oder mehreren erfindungsgemäßen zweiteiligen Unterkieferprotrusionsschienen (1);
(B) einer Anpassvorrichtung (14) für das Oberteil der zweiteiligen Unterkieferprotrusionsschienen, welche in ihrer Größe und Form so ausgestaltet ist, dass sie die Außenseite der Bodenfläche der Oberkieferschale (3a) unterstützt und mit einer, an ihrer Oberfläche angebrachten Vorrichtung (14a), der in die nach vorne geöffnete Führungsschiene (7) der Oberkiefer-Schale (3a) eingreift; und
(C) gegebenenfalls eine Gebrauchsanleitung, worin das Einformen, Adjustieren und Reinigen der zweiteiligen Unterkieferprotrusionsschienen erläutert werden.

Die erfindungsgemäße universelle Unterkieferprotrusionsschiene besitzt den Vorteil, dass sie vergleichbar einer dentalen Schiene sehr fest auf beiden Kiefern sitzt bzw. haftet, da aufgrund der Materialbeschaffenheit der thermoplastischen Schienenfüllung problemlos eine sehr tiefe und gleichmäßige Impression sämtlicher Zähne erreicht wird. Begünstigt wird der feste Sitz auch durch die feste Einfassung des Thermoplasten durch die festen Standardschiene passend für fast sämtliche Kieferformationen. Des weiteren ist in höchst vorteilhafter Weise eine individuelle stufenlose Einstellung des Unterkiefervorschubs möglich. Durch die spezielle Konstruktion der Schiene wird erreicht, dass diese sehr zierlich ist und nach Anpassung der Abstand der oberen und unteren Frontzähne mit weniger als 10 mm nur sehr klein ist, was sich sehr positiv auf Tragekomfort und Akzeptanz auswirkt und auch darauf, dass die Schiene gleichzeitig für unterschiedlich große Kieferformationen geeignet ist.

### KURZE BESCHREIBUNG DER ABBILDUNGEN

Figur 1 zeigt eine perspektivische Ansicht von schräg oben und vorne einer Ausführungsform der erfindungsgemäßen universellen Protrusionsschiene;
Figur 2 zeigt einen Längsschnitt durch diese Protrusionsschiene entlang der Stellschraube;
Figur 3 zeigt eine perspektivische Ansicht des Oberteils jeweils von schräg oben und von schräg unten;
Figur 4 zeigt einen Längsschnitt durch das Oberteil;
Figur 5 zeigt eine Aufsicht des Oberteils von oben;
Figur 6 zeigt eine Vorderansicht des Oberteils;
Figur 7 zeigt eine perspektivische Ansicht des Unterteils jeweils von schräg oben und von schräg unten;
Figur 8 zeigt einen Längsschnitt durch das Unterteil;
Figur 9 zeigt eine Aufsicht des Unterteils von oben;
Figur 10 zeigt eine Vorderansicht des Unterteils;
Figur 11 zeigt eine geeigneten Anpassvorrichtung für das Oberteil;
Figur 12 zeigt eine weitere Ausführungsform der erfindungsgemäßen, individuell angepassten Protrusionsschiene seitlich von vorne;
Die Figuren 13a, 13b und 13c zeigen alternative Ausführungsformen von Verbindungselementen der individuell angepasste Protrusionsschiene.

### DETAILLIERTE BESCHREIBUG DER ERFINDUNG

Die erfindungsgemäße Protrusionsschiene lässt sich aus bekannten Materialien entweder universell massengefertigt im Spritzgussverfahren oder in einer individuellen Variante durch Befestigen eines geeigneten Verbindungselementes an im Dentallabor gefertigte Schalen für Ober- und Unterkiefer herstellen.

Der Begriff "Protrusionsschiene" bzw." Unterkieferprotrusionsschiene" wie er vor- und nachstehend verwendet wird bezeichnet eine dentale Vorrichtung, welche es erlaubt, den Unterkiefer gegenüber dem Oberkiefer in eine leicht nach vorne gelagerte Position zu verschieben und dadurch den Querschnitt der oberen Atemwege zu vergrößern. Dies führt zu einer Reduktion des Schnarchens und von Atemaussetzern infolge eine obstruktiven Schlafapnoe.

Der Begriff "thermoplastisches Füllmaterial" wie er vor- und nachstehend verwendet wird bezeichnet ein Material, das sich in der Wärme, vorzugsweise unterhalb von 60 °C, vorzugsweise zwischen 40 und 50 °C plastisch verformen lässt und sich eng an eine vorgegebene Form anschmiegt und danach beim Abkühlen diese besagte Form beibehält. Geeignete Materialien sind Polymere und Kopolymere oder Gemische davon aus der Gruppe der Polyethylene (PE), Polyvinylacetate (PVA), Acrylate und Methacrylate, bevorzugt Kopolymere aus PE und PVA, wie sie zum Beispiel unter der Marke Elvax® der Firma DuPont erhältlich sind. Bevorzugt sind solche PE-PVA Kopolymere, deren PVA Gehalt von 20 bis 40, insbesondere 25 bis 35 % beträgt

Der Begriff "formgebende und biegesteife Schale" wie er vor- und nachstehend verwendet wird bezeichnet sowohl einen "U- bzw. hufeisenförmigen" Formkörper, der eine an den Enden offene oder geschlossene Schale oder Wanne ausbildet, als auch durch ein Ober- bzw. Unterteil einer durch individuelle Anpassung hergestellten Dentalschiene. In der Regel besteht dieser Formkörper aus einem unter physiologischen Bedingungen inerten und stabilen Material wie zum Beispiel duroplastischen Kunststoffen, wie zum Beispiel Polytetrafluoroethylen (PTFE, Teflon®), oder Polykarbonaten oder aus Elastomeren wie zum Beispiel Polyacrylaten.

Der Ausdruck "eine nach vorne geöffnete Führungsschiene" wie er vor- und nachstehend verwendet wird bezeichnet eine "C-Schiene", die den Schraubenkopf umfasst und ein Lösen der Verbindung zwischen Stellschraube und der Schraubenführung der unteren Schale in Längsrichtung nach vorne verhindert, aber seitliche Bewegungen des Unterkiefers zulässt. Diese "C-Schiene" ist an beiden seitlichen Enden geöffnet, so dass der Kopf der Stellschraube leicht von der Seite eingeschoben werden kann.

In der Regel besteht die "C-Schiene" aus dem gleichen Material wie die biegestabile Schale und ist fest mit der Unterseite der Schale verbunden oder sie besteht in der individuellen Variante aus einem inerten, stabilen Metall und befindet sich auf einer Bodenplatte aus Metall, die vorzugsweise eine oder mehrere Aussparungen aufweist Diese Ausführungsform eignet sich insbesondere zum Einbetten in eine individuell auf Grund eines Abdruckes des Gebisses des Patienten hergestellten Dentalschiene.

Der Begriff "Stellschraube" wie er vor- und nachstehend verwendet wird bezeichnet ein stufenloses Verbindungselement, welches in der Schraubenführung ( 8) stufenlos parallel zum Bodenteil des Unterteils verstellt werden kann. Der Schraubenkopf seinerseits sitzt nahezu unbeweglich fest in der "C-Schiene". In der Regel besteht diese Stellschraube aus einem unter physiologischen Bedingungen inerten und stabilen Material wie zum Beispiel Metallen wie Titan oder Edelstahl, keramischen Materialien oder duroplastischen Kunststoffen.

In der Regel besteht die "Schraubenführung" aus dem gleichen Material wie die biegestabile Schale und ist fest mit der Unterseite der Schale fest verbunden oder sie besteht in der individuellen Ausführungsform aus einem inerten, stabilen Metall und befindet sich auf einer Bodenplatte aus Metall, die eine oder mehrere Aussparungen aufweist Diese Ausführungsform eignet sich insbesondere zum Einbetten in eine individuell auf Grund eines Abdruckes des Gebisses des Patienten hergestellten Dentalschiene

Ausführungsform eignet sich insbesondere zum Einbetten in eine individuell auf Grund eines Abdruckes des Gebisses des Patienten hergestellten Dentalschiene

Die vor- und nachstehend verwendeten Angaben hinsichtlich der Geometrie oder räumlichen Anordnung der Protrusionsschiene oder von Teilen davon orientieren sich an den Gegebenheiten der Protrusionsschiene bei ihrer sinngemäßen Verwendung, d.h. nach Einsetzen derselben auf die obere und untere Zahnreihe der betroffenen Person: "in Längsrichtung" bedeutet in Richtung der Mundöffnung ("anterior") oder des Rachens ("posterior"); "nach vorne" (anterior) bedeutet in Richtung der Mundöffnung; "mittig" bedeutet im Bereich der Schneidezähne; "hinten" bedeutet im Bereich der Backenzähne.

Vorteilhafte Ausgestaltungen der Erfindung sind solche universellen Unterkieferprotrusionsschienen, wobei
(a) das Füllmaterial (4, 5) aus einem oder mehreren Kopolymeren aus Polyethylen und Polyvinylacetat besteht, insbesondere worin das Füllmaterial aus einem thermoplastischen Kopolymeren oder einer Mischung von zwei oder mehreren thermoplastischen Kopolymeren besteht, welche ihrerseits aus Polyethylen und Polyvinylacetat bestehen, und die Erweichungstemperatur des resultierenden Kunststoffes bei einer Temperatur von 40 bis 50° C, vorzugsweise bei etwa 44° C liegt;
(b) in das Füllmaterial (4) der Oberschale zur Aufnahme der Zähne eine Orientierungsmulde (13) eingeformt ist;
(c) die formgebende und biegesteife Schale (2a, 3a) aus Polycarbonat besteht;
(d) der Abstand zwischen der Innenseite des Bodens der Oberkieferschale (23) und der Innenseite des Bodens der Unterkieferschale (22), im zusammengesetzten Zustand weniger als 10 mm, vorzugsweise von 6 bis 9 mm, insbesondere etwa 8 mm beträgt.; besonders bevorzugt ist ein Abstand der Zähne im Frontzahnbereich von etwa. 8,0 bis. 8,4 mm bei angelegter Schiene erreicht. Dies führt im Regelfall zu einem leichterem und dabei vollständigerem Schließen des Mundes auch bei kleinen Kiefergrößen ohne Muskelanspannung. Dadurch resultieren höherer Tragekomfort und damit auch eine bessere Akzeptanz (Compliance).
(e) ihre Höhe im zusammengesetzten Zustand weniger als 30 mm, vorzugsweise von 20 bis 28 mm, insbesondere etwa 27 mm beträgt;
(f) ihre Gesamtlänge, d.h. der Abstand zwischen dem vordersten Teil zu der von den beiden Schenkel gebildeten Mitte, im zusammengesetzten Zustand weniger als 50 mm, vorzugsweise von 42 bis 48 mm, insbesondere etwa 45 mm beträgt;
(g) die beiden Schalen (2a, 3a) so miteinander verbunden sind, dass sie in der Längsrichtung stufenlos um bis zu 14 mm, vorzugsweise > 0 bis 12 mm gegeneinander verstellbar sind. Besonders vorzugsweise wird die Unterkieferprotrusionsschiene zusammen mit mindestens drei Stellschrauben unterschiedlicher Länge zur Verfügung gestellt, wobei vorzugsweise eine Stellschraube eine Länge von bis zu 14 mm, insbesondere etwa 12 mm aufweist und die andere Stellschraube eine Länge von bis zu 18 mm, insbesondere etwa 16 mm aufweist und eine dritte Stellschraube eine Länge von bis zu 22 mm, insbesondere etwa 20 mm aufweist.

Die Herstellung der erfindungsgemäßen universellen Unterkieferprotrusionsschiene an sich ist einfach:
(a) man formt die formgebenden und biegesteifen Schalen (2a, 3 a) mit jeweils auf den Außenseiten der Bodenflächen vorgeformten Befestigungselementen im Spritzgußverfahren;
(b) füllt die Schalen mit einem thermoplastischen Füllmaterial (4, 5) ebenfalls per Spritzgussverfahren bei Massenfertigung bzw. in besonderen Einzelfällen auch manuell hergestellt; und
(c) verbindet die vorgeformten Befestigungselemente.

Das verwendete Kopolymer kann, abgesehen von der üblichen Massenfertigung im Spritzgussverfahren, auch in besonderen Einzelfällen nach Erhitzen in Granulatform im Wasserbad manuell zu einer homogenen plastischen Masse geformt und dann in leere ungefüillte Schalen eingedrückt werden. Wenn man anschließend diese Schalen mit ihrer Füllung in heißem, vorzugsweise kochendem Wasser etwa 2 bis 4 Minuten erhitzt und nach kurzem Abkühlen die plastische Masse in den Schalen gleichmäßig verteilt und sodann aushärten lässt, kann man so eine voll funktionsfähige, erfindungsgemäße Unterkieferprotrusionsschiene herstellen. Die polymere Masse haftet absolut fest auf der Kontaktfläche aus Polycarbonat. Falls einem Arzt oder einem Patienten beim ersten Versuch die Anpassung durch Einbeißen an der falschen Stelle misslingen sollte, kann auf diese Art und Weise die Schiene sehr einfach wieder in den Neuzustand zurückversetzt werden.

Sollte weiterhin nach langer Tragezeit über viele Monate die Haftung der Schiene an Zähnen und Kiefern nachlassen, kann man so ebenfalls problemlos die ursprüngliche Schienenfüllung vollständig wiederherstellen und dann die Anpassung erneut vornehmen. Die erfindungsgemäße Unterkieferprotrusionsschiene kann somit gegebenenfalls nach zwischenzeitlichen Reparaturen über einen sehr langen Zeitraum verwendet werden. Ein weiterer Gegenstand der Erfindung ist somit ein Set zum Herstellen bzw. Wiederherstellen einer erfindungsgemäßen Unterkieferprotrusionsschiene gemäß Anspruch 10.

Weiterhin kann in an sich bekannter Weise auf Grund eines Zahnabdruckes des Patienten an einer individuell im Dentallabor gefertigten Dentalschiene an den Unterseiten von Ober- und Unterschale jeweils ein Verbindungselement, bestehend aus einer "C-Schiene" und einer Schraubenführung, hergestellt aus Edelstahl oder einem physiologisch inertem Metall wie z.B. Titan, angebracht werden.

Diese Ausführungsform kann hergestellt werden, in dem in an sich bekannter Weise ausgehend von einem Gebissabdruck des Ober- und Unterkiefers eines Patienten, vorzugsweise in einem Dentallabor eine Dentalschiene gefertigt wird. Dabei werden Alginatabrücke vom einem Zahnarzt genommen und von diesem die Unterkieferprotrusion vorgegeben (so genannte Konstruktionsbissnahme unter Verwendung einer Bissgabel zur Fixierung einer leichten Protrusion des Unterkiefers).

Im Dentallabor wird sodann ein Gipsmodell konstruiert und mit Hilfe der Protrusionsbissnahme einartikuliert. Mit Hilfe der Druckfomtechnik werden nach Modelldublizierung hartelastische Schalen für Ober- und Unterkiefer als Konstruktionsbasis für die individuelle Unterkieferprotrusionsschiene tiefgezogen. Für den Herstellungsprozess können alle gängigen handelsüblichen Tiefziehplatten in unterschiedlichen Dicken verwendet werden, zB. Erkoloc Pro (Erkodent GmbH) in einer Dicke von 2 bis 3 mm, Duran (Scheu Dental GmbH) in einer Dicke von 2,0 mm u.v.a. Für den Tiefziehvorgang können ebenfalls marktgängige Geräte verwendet werden wie z.B. die Geräte BIOSTAR^{®} oder MINISTAR^{®} der Fa. Scheu Dental GmbH oder ERKOPRESS^{®} der Erkodent GmbH.

Nach Erkalten der tiefgezogenen Schalen für Ober- und Unterkiefer kann Acrylharz auf der Oberfläche verteilt werden. Dadurch werden die thermoformen Schalen fester und lassen sich besser bearbeiten (polieren). In der Regel wird im Frontzahnbereich kein Acrylat aufgetragen. Danach wird die so erhaltene Rohform vom Modell entfernt, zugeschnitten und poliert.

Anschließend wird an den Stellen im Frontzahnbereich, wo das Verbindungselement fixiert werden soll, ein Acrylatkleber aufgetragen. Die untere Bodenplatte mit der Schraubenführung wird mir Acrylatkleber auf der Unterseite der Unterkieferschiene fixiert und die Befestigungslöcher der Bodenplatte mit glasklar, kalt polymerisierendem Kunststoff bedeckt. Im Bereich der 6er- und 7er Zähne kann Kunststoff aufgebracht werden, um -falls vom Zahnarzt vorgegeben- eine distale Abstützung zu gewährleisten..

Nach dem gleichen Verfahren wird die obere Bodenplatte mit der "C-Schiene" mit Acrylatkleber im Frontzahnbereich der Oberkieferschiene fixiert.

Nach Aushärten des Acrylatklebers werden die Schienen geschliffen und poliert. Die beiden Schalen für Ober- und Unterkiefer können sodann über eine Stellschraube miteinander verbunden werden.

Die Herstellung der Schraubenführung und der "C-Schiene" erfolgt in an sich bekannter Weise. Man kann entweder die entsprechenden Formteile aus einem Werksstoffblock fräsen, durch Falztechniken, gegebenenfalls unter Erwärmen aus Platten bzw. Blechen formen, durch Verschweißen oder Löten, insbesondere durch Schweißen mit Laser mehrerer vorgefertigter Bauteile, insbesondere von einer Bodenplatte mit einer "C-Schiene" oder einer Bodenplatte mit einer Schraubenhülse oder Schraubenbuchse, oder sofern diese aus Duroplasten bestehen, auch im Spritzgussverfahren herstellen.

Ein weiterer Gegenstand der Erfindung ist somit ein gebrauchsfertiges Set zum Herstellen eines Verbindungselementes für eine erfindungsgemäße Unterkieferprotrusionsschiene gemäß Auspruch 11.

Ausführungsbeispiele der Erfindung sind in den Zeichnungen dargestellt und werden im folgenden näher beschrieben.

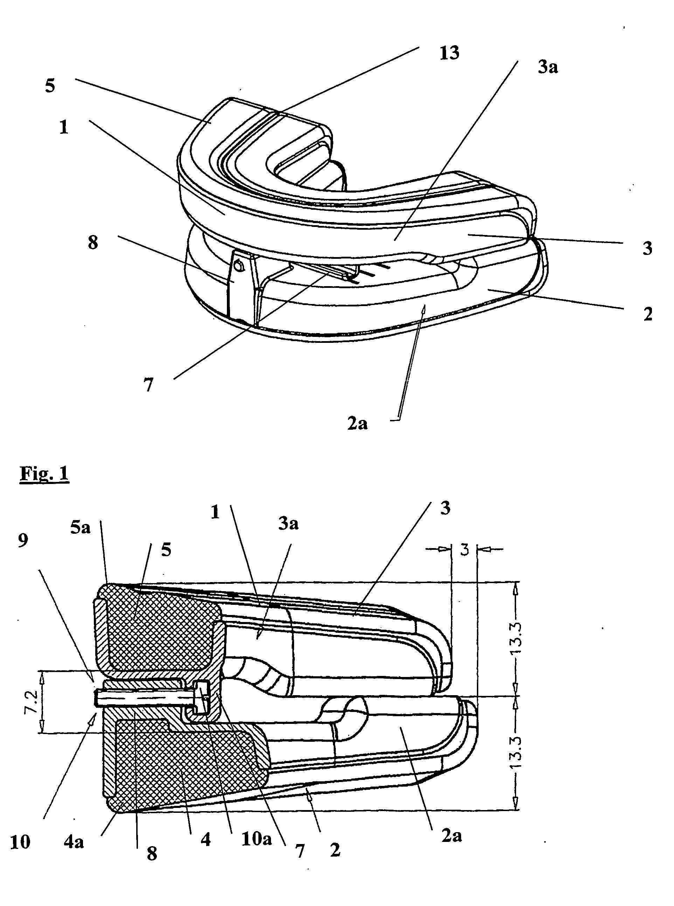

Figur 1 zeigt eine perspektivische Ansicht von schräg oben und vorne einer Ausführungsform der erfindungsgemäßen universellen Protrusionsschiene (1) gebildet aus einem Unterteil (2) und einem Oberteil (3), die jeweils aus einer Schale (2a, 3a) und einem Füllmaterial (4, 5) gebildet werden. Das Füllmaterial (4) des Unterteils ist in Fig. 1 nicht zu sehen, jedoch erkennt man deutlich die Mulde (13) im Füllmaterial (5) des Oberteils, welche zur besseren Orientierung der Zähne beim Einsetzen der Protrusionsschiene dient. Weiterhin weist das Unterteil (2) der Protrusionsschiene (1) im Vorderbereich eine Schraubenführung ( 8) mit einem Gewinde (9) zur Aufnahme einer in Fig. 1 nicht zu erkennenden Stellschraube, deren Kopf in die Führungsschiene ("C-Schiene") (7) eingreift.

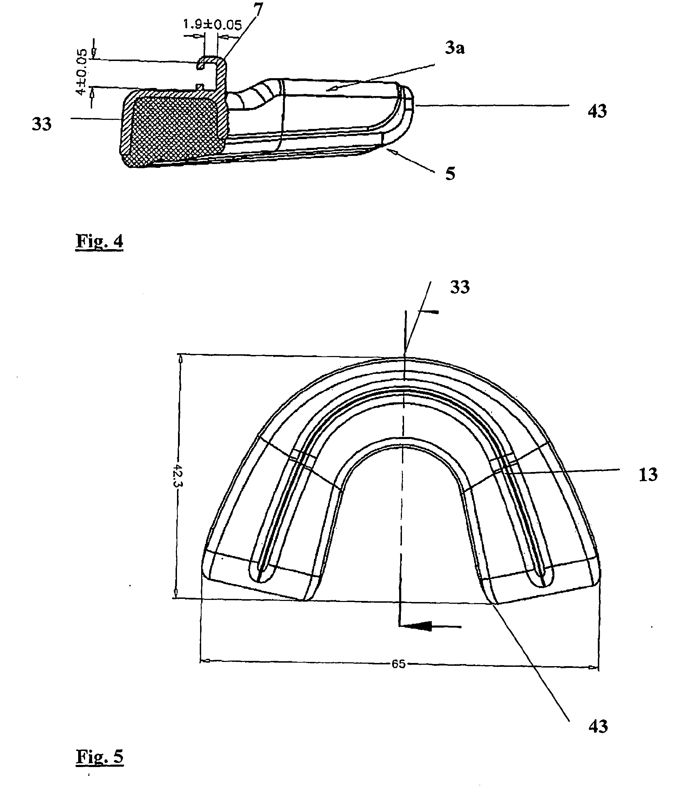

Figur 2 zeigt einen Längsschnitt durch diese Protrusionsschiene (1) entlang der Stellschraube (10) gebildet aus einem Unterteil (2) und einem Oberteil (3), die jeweils aus einer Schale (2a, 3a) und einem Füllmaterial (4, 5) gebildet werden. Wie aus den Fig. 2, 4 und 8 zu erkennen ist, überragt das thermoplastische Material (4, 5) jeweils die Höhe der biegefesten Schalen, vorzugsweise um 0,5 bis 3 mm, insbesondere um 1 bis 1,5 mm (4a, 5a). Dies führt zu einer erhöhten Haftung der Zähne und gleichzeitig zu einem Schutz des Zahnfleisches gegenüber Verletzung durch die Kanten der biegefesten Schalen.

Die Stellschraube (10) befindet sich im voll eingeschraubten Zustand in der Schraubenführung (8), ihr Kopf (10a) ist von der "C-Schiene" (7) umfasst.

Weiterhin ist deutlich zu erkennen, dass die Außenwangen (32, 33) der beiden Schalen im vorderen Bereich höher sind als die jeweiligen Innenwangen (42, 43). Die vordere Begrenzung der Schraubenführung (8) schließt bündig an die Außenwange (42) des Unterteils an. Die Länge des Gewindes in der Schraubenführung (8) beträgt 6 bis 12 mm, vorzugsweise 8 bis 10 mm, insbesondere etwa 9 mm.

Der Abstand zwischen den inneren Böden der beiden Schalen im Frontzahnbereich ("Zahnabstand") beträgt weniger als 10 mm, vorzugsweise 6 bis 9 mm, insbesondere etwa 8 mm. Im Bereich der Schneidezähne weist die Protrusionsschiene vor Anpassung an das Gebiss eine Gesamthöhe von weniger als 30 mm auf, vorzugsweise 24 bis 28 mm, insbesondere etwa 26.5 mm auf.

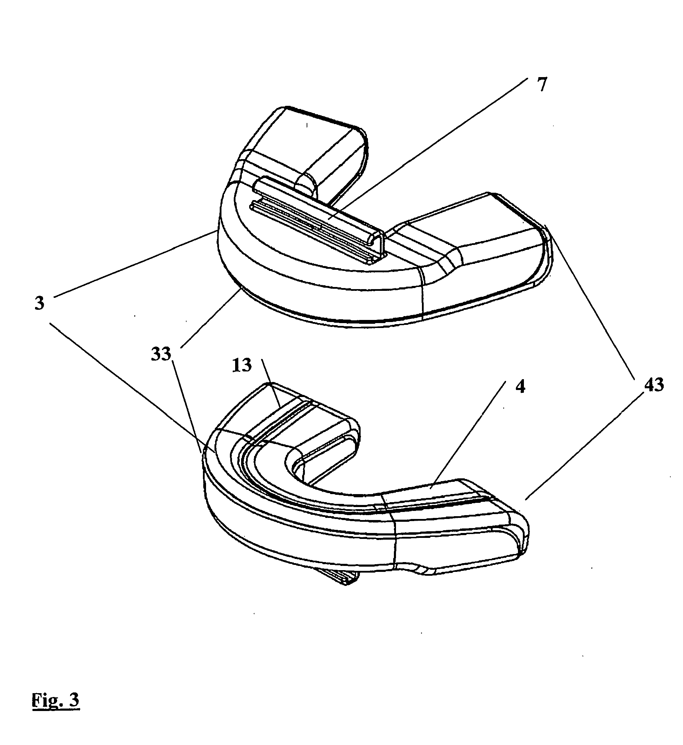

Die in den Figuren 3 bis 6 dargestellten Detailansichten des Oberteils (3) verdeutlichen den Aufbau und die Funktion dieses Teils der erfindungsgemäßen Protrusionsschiene. Die Länge der nach vorne geöffneten Führungsschiene (7) beträgt weniger als 35 mm, vorzugweise 26 bis 30 mm, insbesondere 28 mm.

Die Länge der Führungsschiene lässt eine laterale Bewegung des Unterkiefers zu, ohne dass es zu einer Lösung der Verbindung beider Teile kommt.

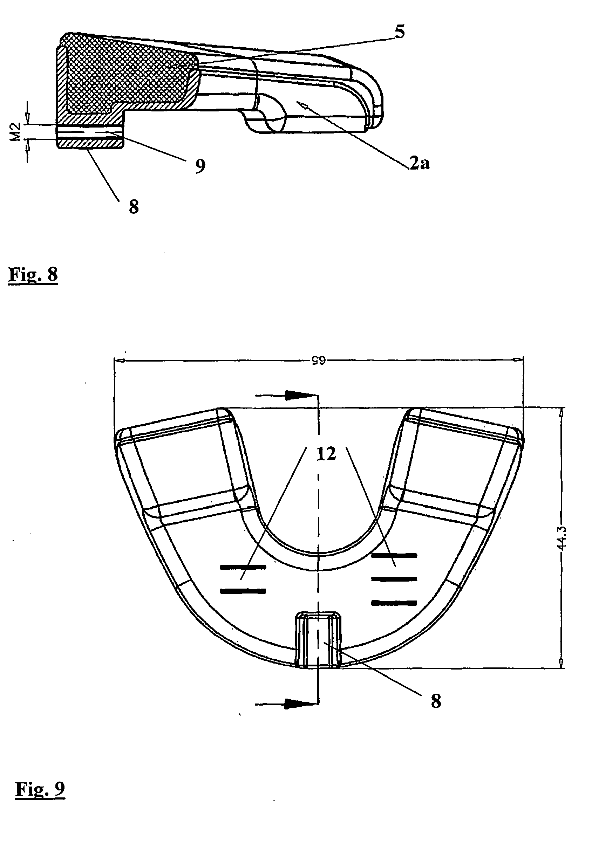

Der Abstand der beiden Außenwangen am hinteren Ende des Oberteils (maximale Breite der Unterkieferschiene) beträgt 60 bis 70 mm, vorzugsweise 62 bis 68 mm, insbesondere etwa 65 mm. Der Abstand zwischen der Außenwange (33) im Bereich der Schneidezähne und einer gedachten Linie zwischen den hinteren Enden der Innenwange (43) (maximale Länge der Unterkieferschiene) beträgt 43 bis 46 mm, insbesondere etwa 44,3 mm (vgl. Fig. 9). Die Gesamthöhe der Führungsschiene (7) beträgt 5 bis 7 mm, insbesondere etwa 6 mm. Die lichte Höhe der Führungsschiene beträgt 3,8 bis 4,2 mm, insbesondere etwa 4 mm. Die lichte Tiefe der Führungsschiene beträgt 1,8 bis 2,0 mm, insbesondere etwa 1,9 mm. Zur Herstellung der biegestabilen Oberschale (3a) werden 3,0 bis 4,0 cm³ Polycarbonat verwendet.

Zur Herstellung der Füllung der Oberschale werden 10,0 bis 12,0 cm³ eines Copolymers aus PE und PVA verwendet.

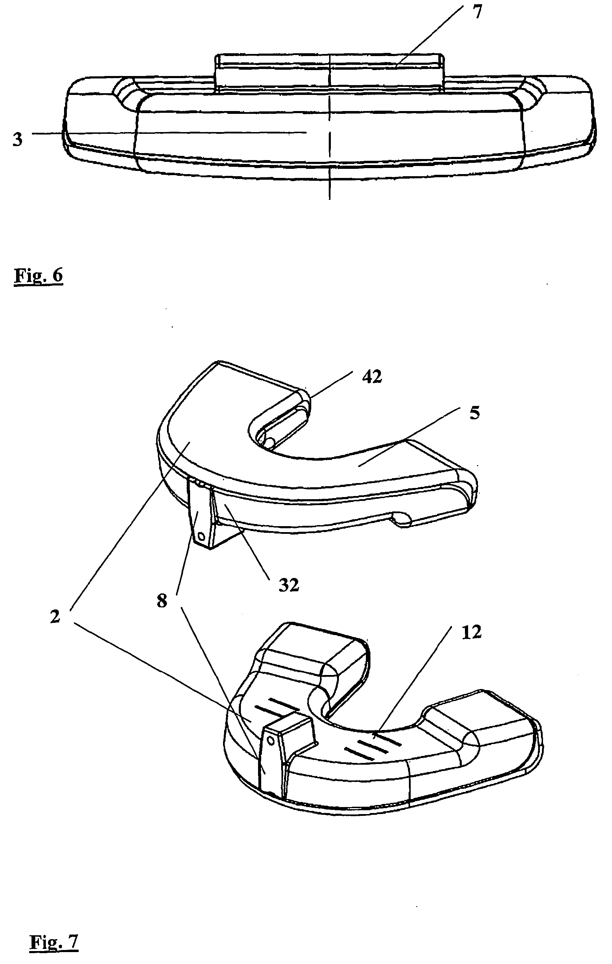

Die in den Figuren 7 bis 10 dargestellten Detailansichten des Unterteils (2) verdeutlichen den Aufbau und die Funktion dieses Teils der erfindungsgemäßen Protrusionsschiene. Die Breite der Schraubenführung (8) senkrecht zur Ausrichtung des Gewindes beträgt 4 bis 9 mm, insbesondere etwa 6 mm. Die Länge der Schraubenführung (8) parallel zur Ausrichtung des Gewindes beträgt 7 bis 11 mm, insbesondere etwa 9 mm. Die Höhe der Schraubenführung (8) beträgt 4 bis 6 mm, insbesondere etwa 5 mm.

Die angegebene Länge Breite und Höhe der Schraubenführung (8) ermöglichen einen sicheren Halt der Stellschraube in dem Gewinde und erlauben es zudem, einen geringen Abstand zwischen den Schneidezähnen des Ober- und Unterkiefers einzustellen, was zu erhöhtem Tragekomfort und einer besseren Akzeptanz führt.

Der Abstand der beiden Außenwangen am hinteren Ende des Unterteils (maximale Breite der Oberkieferschiene) beträgt 60 bis 70 mm, vorzugsweise 62 bis 68 mm, insbesondere etwa 65 mm. Der Abstand zwischen der Außenwange (32) im Bereich der Schneidezähne und einer gedachten Linie zwischen den hinteren Enden der Innenwange (42) (maximale Länge der Oberkieferschiene) beträgt 40 bis 44 mm, insbesondere etwa 42,5 mm. Zur Herstellung der biegestabilen Unterschale wurden 2,5 bis 3,5 cm³ Polycarbonat verwendet. Zur Herstellung der Füllung der Unterschale (2a) wurden 10,0 bis 12,0 cm³ eines Copolymers aus PE und PVA verwendet.

In Figur 9 kann man rechts und links der Schraubenführung (8) zwei bzw. drei gerade Linien erkennen. Diese dienen als Skala (12) um eine reproduzierbare Verstellung der Unterkieferschiene mittels der Stellschraube zu gewährleisten.

Das Anpassen der erfindungsgemäßen universellen Protrusionsschiene an den Kiefer der betroffenen Person ist einfach. Die Anpassung kann im Regelfall von jedem Arzt gleich welcher Fachrichtung, dessen instruiertem Personal oder zumeist auch von der betroffenen Person alleine mit Hilfe eines Spiegels durchgeführt werden.

Bei Vorliegen von Zahnfehlstellungen, anamnestischen Kiefergelenksbeschwerden oder Zahnerkrankungen wie z.B. Parodontose u.a. ist es ratsam, vor Anpassung einen Zahnarzt zu konsultieren..

Bei der Anpassung der universellen Schiene erwärmt man zuerst das Oberteil (3) auf eine Temperatur oberhalb der Erweichungstemperatur des Füllmaterials (4), vorzugsweise in einem Wasserbad bei einer Wassertemperatur von oberhalb 50 °C. Danach passt man das Oberteil an die Zahnreihe des Oberkiefers an, indem man die Zähne in das weiche, plastische und noch warme Füllmaterial gleichmäßig tief eindrückt. Anschließend lässt man das Füllmaterial kurz ca. 30 - 60 Sekunden im Mund abkühlen und dann endgültig in einem kalten Wasserbad aushärten.

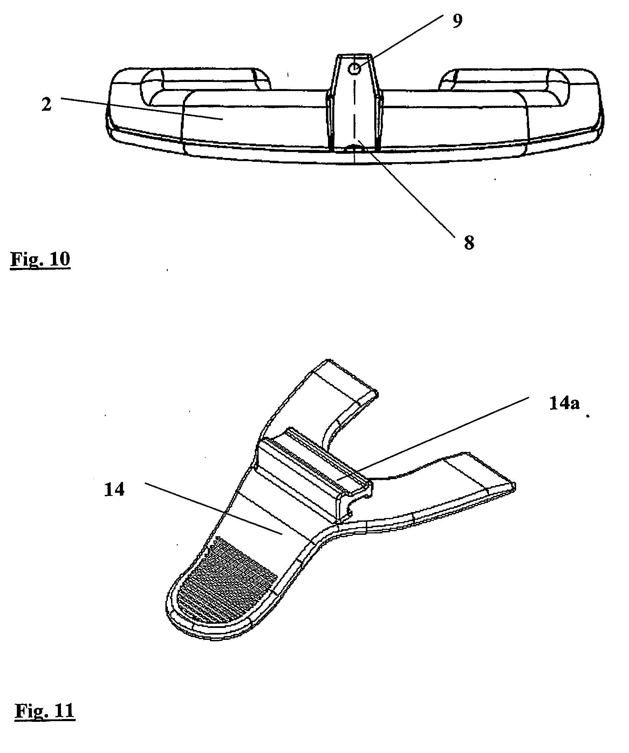

Hilfsweise kann die Anpassung des Oberteils (3) mit einer in Figur 11 gezeigten Anpassvorrichtung (14) erfolgen.

Danach erwärmt man das Unterteil (2) entsprechend und verbindet es mit dem bereits angepassten Oberteil mit Hilfe der Stellschraube, die man zunächst für den Zweck der Anpassung bis zum Anschlag wie in Fig. 2 gezeigt einschraubt, setzt das ausgeformte Oberteil in den Oberkiefer ein und drückt vorsichtig die Zähne des Unterkiefers in das weiche Füllmaterial (5) des Unterteils ein. Nach Aushärten wird die Stellschraube in die für die jeweilige Person geeignete Position gedreht.

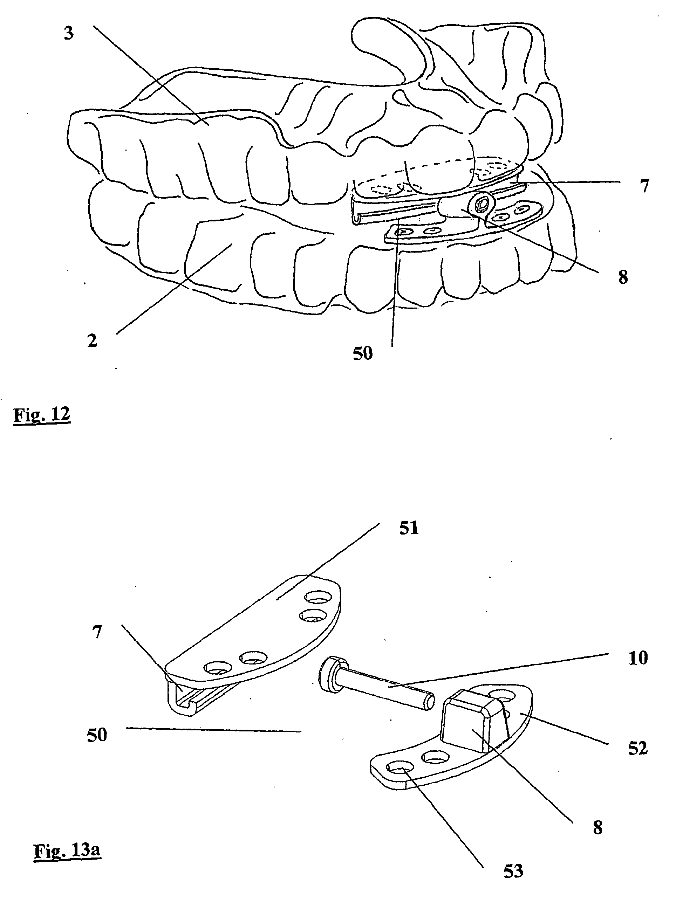

In Fig. 12 zeigt eine Darstellung einer weiteren Ausführungsform der Protrusionsschiene (1), bestehend aus einem Unterteil (2) und einem Oberteil (3), welche individuell an den Unter- bzw. Oberkiefer eines Patienten angepasst wurden und in die nachträglich ein Verbindungselement (50) bestehend aus einer "C-Schiene" (7) und eine Schraubenführung (8) eingebettet wurden.

Die Stellschraube (10) befindet sich im eingeschraubten Zustand in der Schraubenführung (8), ihr Kopf (10a) ist von der "C-Schiene" (7) umfasst. Durch eine zusätzliche Kontermutter kann die Protrusion in einer vorher festgelegten Position fixiert werden.

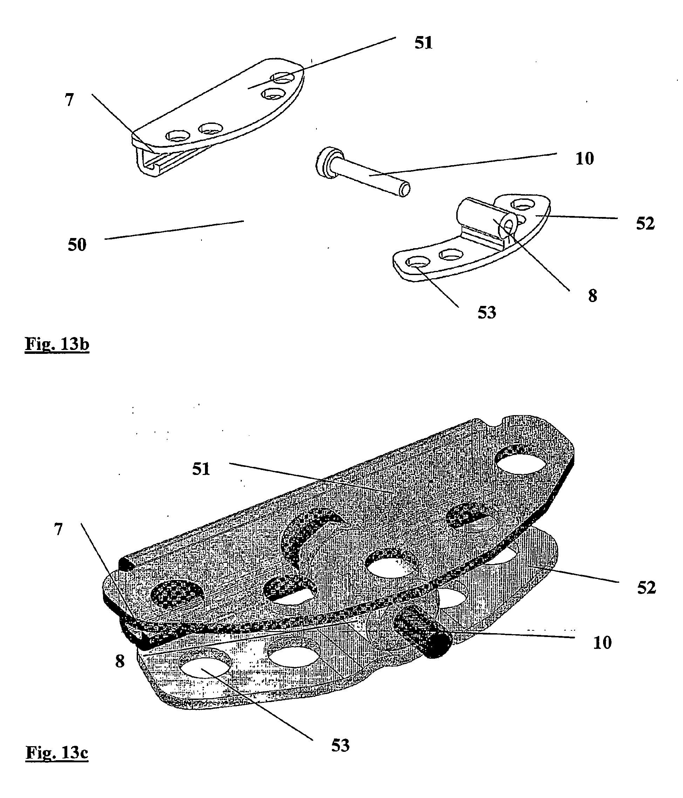

Die Fig. 13a, 13b und 13c zeigen Details von alternativen Ausführungsformen der Verbindungselemente (50) mit einer senkrecht zu einer Bodenplatte (51) angeordneten "C-Schiene" (7) und einer ebenfalls senkrecht zu einer Bodenplatte (52) angeordneten Schraubenführung (8). Die Formen beider Bodenplatten sind an die Form Bodenfläche der jeweils mit ihr zu verbindenden Schale angepasst und weisen eine oder mehrere Aussparungen (53) auf. Während die einzelnen Komponenten der Verbindungselemente der Fig. 13 a und 13b getrennt dargestellt sind, zeigt Fig. 13c ein erfindungsgemäßes Verbindungselement, worin die Schraube (10) mit der Schraubenführung (8) verschraubt ist und der Schraubenkopf in die "C-Schiene" (7) eingreift.

Das Verbindungselement der Fig. 13a wurde durch Fräsen aus 2 Metallböcken hergestellt. Das Verbindungselement der Fig. 13b wurde durch Biege- und Falztechniken aus entsprechend vorgeformten Metallblechen erhalten. Bei dem Verbindungselement der Figur 13c wurden jeweils eine "C-Schiene" (7) und eine Schraubenbuchse (8) durch Laserschweißen auf der zugehörigen Bodenplatte (51) bzw. (52) befestigt.

Die bezüglich der Figuren 3 bis 10 angegebenen bevorzugten Abmessungen der Führungsschiene (7) und der Schraubenführung (8) treffen auch auf die Führungsschiene (7) und Schraubenführung (8) der Figuren 12, 13a, 13b und 13c zu.

Die erfindungsgemäße universelle Unterkieferprotrusionsschiene beseitigt oder reduziert Schnarchen und Atemaussetzer infolge einer obstruktiven Schlafapnoe. Durch Ihre zierliche Größe weist sie einen hohen Tragekomfort auf. Sie vermittelt einen festen, sicheren Halt für die umfassten Zähne, kann stufenlos hinsichtlich des Unterkiefervorschubs an die Bedürfnisse des Patienten angepasst werden, schützt die Zähne gegen nächtliches Zähneknirschen und das Zahnfleisch vor Verletzungen.

Die mit Hilfe der Verbindungselemente im Dentallabor individuell gefertigte Unterkieferprotrusionsschiene ist aufgrund der sehr dünnen Tiefziehplatten noch filigraner und weniger voluminös gearbeitet. Die Zunge des Patienten hat daher in der Mundhöhle noch mehr Platz, was einem im Vergleich zur universellen Schiene verbessertem Tragekomfort zugute kommt. Abhängig von der Strapazierfähigkeit der verwendeten Tiefziehplatten, die stark von deren chemischer Zusammensetzung und Dicke abhängt, ist die Lebensdauer der individuellen Schiene größer als die der universellen Schiene. Die individuelle Protrusionsschiene erscheint daher im Vergleich zur universellen , massengefertigten und preiswerten Schiene eher geeignet für die langjährige Dauertherapie. Da unabhängig von der Herstellungsweise bei keiner Unterkieferprotrusionsschiene Wirkung und Akzeptanz durch den Patienten vorhergesagt werden können, empfiehlt sich aus Kostengründen zunächst stets der Einsatz der preiswerteren universellen Schiene als Erstversorgungsschiene. Bei erwiesener Wirksamkeit und Akzeptanz durch den Patienten sollte die universelle dann z.B. nach einem bis zwei Jahren zwecks langfristiger Dauerbehandlung durch die individuell gefertigte Unterkieferprotrusionsschiene ersetzt werden.

## Patentansprüche

1. Zweiteilige Unterkieferprotrusionsschiene (1 ) zur Verhinderung von Schnarchen und/oder von obstruktiver Schlafapnoe, umfassend ein Unter- (2) und ein Oberteil (3), aus einer jeweils in Gebrauch zum Unter- bzw. Oberkiefer geöffneten, formgebenden und biegesteifen Schale (2a, 3a), wobei jede schale eine Bodenfläche mit jeweils einer Außenseite und eine Innenseite aufweist, **dadurch gekennzeichnet, dass** die Außenseite der Bodenfläche der Unterkiefer-Schale (2a) mittig eine Scbraubenföhrung (8) mit einem parallel zur Bodenfläche verlaufenden Gewinde aufweist, und die Außenseite der Bodenfläche der Oberkieferschale (3 a) mittig eine nach vorne geöffnete Führungsschiene (7) zur Aufnahme des Schraubenkopfes einer in dem Gewinde befindlichen Stellschraube (10) aufweist, wodurch die beiden Schalen (2a, 3a) so miteinander verbunden werden können, dass sie in der Längsrichtung stufenlos gegeneinander verstellbar sind.

2. Unterkieferprotrusionsschiene nach Anspruch 1, **dadurch gekennzeichnet dass** die biegesteifen Schalen (2a, 3 a) ein thermoplastisches Füllmaterial (4, 5) enthalten, welches jeweils zahnspangenartig dem menschlichen Ober- und Unterkiefer nachformbar ist.

3. Unterkieferprotrusionsschiene nach Anspruch 1 oder 2, **dadurch gekennzeichnet, dass** in das Füllmaterial (4) der Oberkiefer-Schale zur Kennzeichnung der Einbissstelle, insbesondere für die Frontzähne, eine Orientierungsmulde (13) eingeformt ist

4. Unterkieferprotrusionsschiene nach Anspruch 1, **dadurch gekennzeichnet, dass** die biegesteifen Schalen (2a, 3a ) mit Hilfe der Druckformtechnik aus handelsüblichen Tiefziehplatten unterschiedlicher chemischer Zusammensetzung und Dicke hergestellt werden und jeweils zahnspangenartig individuell dem menschlichen Ober- und Unterkiefer nachgeformt sind.

5. Unterkieferprotrusionsschiene nach einem der Ansprüche 1 bis 4, **dadurch gekennzeichnet, dass** der Abstand zwischen der Innenseite der Bodenfläche der Oberkiefer-Schale (23) und der Innenseite der Bodenfläche der Unterkiefer-Schale (22), im zusammengesetzten Zustand weniger als 10 mm, vorzugsweise von 6 bis 9 mm, insbesondere etwa 8 mm beträgt.

6. Unterkieferprotrusionsschiene nach einem der Ansprüche 1 bis 5, **dadurch gekennzeichnet, dass** der Abstand der Zähne bei angelegter Schiene im Frontzahnbereich bei etwa 8,0 bis. 8,4 mm liegt.

7. Unterkieferprotrusionsschiene nach einem der Ansprüche 1 bis 6, **dadurch gekennzeichnet, dass** die Bodenfläche der Unterkiefer-Schale (2a) eine Skala (12) zur reproduzierbaren Einstellung der Stellschraube (10) aufweist,

8. Gebrauchsfertiges Set zur Herstellung einer Unterkieferprotrusionsschiene zur Verhinderung von Schnarchen und/oder (obstruktiver) Schlafapnoe nach einem der Ansprüche 1 bis 7 bestehend aus
(A) einem Unter- (2) und einem Oberteil (3), aus einer jeweils in Gebrauch zum Unter- bzw. Oberkiefer geöffneten, formgebenden und biegesteifen Schale (2a, 3a), wobei die Bodenflächen dieser Schalen jeweils eine Außenseite und eine Innenseite aufweisen, und die Außenseite der Bodenfläche der Unterkiefer-Schale (2a) mittig eine Schraubenführung (8) mit einem parallel zur Bodenfläche verlaufenden Gewinde aufweist, und die Außenseite der Bodenfläche der Oberkieferschale (3 a) mittig eine nach vorne geöffnete Führungsschiene (7) zur Aufnahme des Schraubenkopfes einer in das Gewinde schraubbaren Stellschraube (10) aufweiset, wodurch die beiden Schalen (2a, 3a) so miteinander verbunden werden können, dass sie in der Längsrichtung stufenlos gegeneinander verstellbar sind;
(B) zwei oder mehrere Stellschrauben (10) unterschiedlicher Länge,

9. Gebrauchsfertiges Set zur Herstellung einer Unterkieferprotrusionsschene zur Verhinderung von Schnarchen und/oder (obstruktiver) Schlafapnoe nach einem der Ansprüche 1 bis 3 sowie 5 bis 7 bestehend aus
(A) einer oder mehreren zweiteiligen Unterkieferprotrusionsschienen (1) nach einem der Ansprüche 1 bis 3 sowie 5 bis 7;
(B) einer Anpassvorrichtung (14) für das Oberteil der zweiteiligen Unterkieferprotrusionsschienen, welche in ihrer Größe und Form so ausgestaltet ist, dass sie die Außenseite der Bodenfläche der Oberkiefer-Schale (3 a) unterstützt und mit einer, an ihrer Oberfläche angebrachten Vorrichtung in die nach vorne geöffnete Führungsschiene (7) der Oberkiefer-Schale (3a) eingreift; und
(C) gegebenenfalls eine Gebrauchsanleitung, worin das Einformen, Adjustieren und Reinigen der zweiteiligen Unterkieferprotrusionsschienen erläutert werden.

10. Gebrauchsfertiges Set zum Herstellen bzw. Wiederherstellen einer Unterkieferprotrusionsschiene nach einem der Ansprüche 2 bis 3 und 5 bis 7 bestehend aus
(i) einem Unter- (2) und einem Oberteil (3), aus einer jeweils in Gebrauch zum Unter- bzw. Oberkiefer geöffneten, formgebenden und biegesteifen Schale (2a, 3a), wobei die Bodenflächen dieser Schalen jeweils eine Außenseite und eine Innenseite aufweisen, und die Außenseite der Bodenfläche der Unterkiefer-Schale (2a) mittig eine Schraubenführung (8) mit einem parallel zur Bodenfläche verlaufenden Gewinde aufweist, und die Außenseite der Bodenfläche der Oberkiefer-Schale (3 a) mittig eine nach vorne geöffnete Führungsschiene (7) zur Aufnahme des Schraubenkopfes einer in das Gewinde schraubbaren Stellschraube (10) aufweist, wodurch die beiden Schalen (2a, 3a) so miteinander verbunden werden können, dass sie in der Längsrichtung stufenlos gegeneinander verstellbar sind;
(ii) einem zur Herstellung des thermoplastischen Füllmaterials (4, 5) geeigneten Kunststoff;
(iii) einer oder mehreren Stellschrauben (10) zum Verbinden derbeiden Schalen (2a, 3a) ; und
(iv) gegebenenfalls eine Gebrauchsanweisung zur Herstellung bzw. Wiederherstellung der Unterkieferprotrusionsschiene;
wobei (ii) mehr als den zur Herstellung der erfindungsgemäßen Unterkieferprotrusionsschiene benötigten Menge an Kunststoff umfasst.

11. Gebrauchsfertiges Set zum Herstellen einer Unterkieferprotrusionsschiene nach einem der Ansprüche 1 und 4 bis 7 im wesentlichen bestehend aus
(i) einem Unter- (2) und einem Oberteil (3) aus einer jeweils in Gebrauch zum Unter- bzw. Oberkiefer geöffneten, formgebenden und biegesteifen Schale (2a, 3a), die jeweils nach Zahnabdrucknahme aus handelsüblichen polymeren Tiefzitehplatten unterschiedlicher chemischer Zusammensetzung und Plattendicke per Druckformtechnik gefertigt wurden, wobei die Bodenflächen dieser Schalen jeweils eine Außenseite und eine Innenseite aufweisen;
(ii) einem Verbindungselement bestehend aus einer senkrecht zu einer Bodenplatte (51) angeordneten Führungsschiene (7) zur Aufnahme des Schraubenkopfes einer in dem Gewinde schraubbaren Stellschraube (10), einer senkrecht zu einer Bodenplatte (52) angeordneten Schraubenführung (8), wobei die beiden Bodenplatten ein oder mehrere Aussparungen (53) aufweisen und ihre Form an die Außenseiten der Bodenflächen der jeweiligen Außenseite der biegesteifen Schalen (2a, 3a) so angepasst sind, dass sie dort jeweils mittig angebracht werden können; und
(iii) Stellschrauben unterschiedlicher Länge zum Verbinden der individuell gefertigten Schalen für Ober- und Unterkiefer, wobei die im zusammengebauten Zustand nach vorne geöffnete Fübrungsschiene (7) die Aufnahme des Schraubenkopfes einer in dem Gewinde befindlichen Stellschraube ermöglicht und **dadurch** so miteinander verbunden werden können, dass die beiden Schalen (2a, 3a) in der Längsrichtung stufenlos gegeneinander verstellbar sind, und
(iv) gegebenenfalls eine Gebrauchsanweisung zur Herstellung der Unterkieferprotrusionsschiene.

## Claims

1. Two-part mandibular protrusion splint (1) for preventing snoring and/or obstructive sleep apnoea, comprising a lower part (2) and an upper part (3), comprising shaping and flexurally rigid trays (2a, 3a) which are open when applied towards the mandibula and maxilla respectively, wherein each tray exhibits a bottom surface each with an outside and an inside, **characterized in that** the outside of the bottom surface of the mandibular tray (2a) has in the centre a screw guide (8) having a thread running parallel to the bottom surface, and the outside of the bottom surface of the maxillar tray (3a) has in the centre a guide rail (7) which is open towards the front for accommodation of the screw head of an adjustment screw (10) located in the thread, enabling the two trays (2a, 3a) to be connected to one another in such a way that they are continuously adjustable relative to one another in the longitudinal direction.

2. Mandibular protrusion splint according to Claim 1, **characterized in that** the flexurally rigid trays (2a, 3a) contain a thermoplastic filling material (4, 5) which can be shaped in each case in the manner of a dental brace to fit the human maxilla and mandibula.

3. Mandibular protrusion splint according to Claim 1 or 2, **characterized in that** an orientation channel (13) is shaped into the filling material (4) of the maxillar tray in order to indicate the bite point, in particular for the anterior teeth.

4. Mandibular protrusion splint according to Claim 1, **characterized in that** the flexurally rigid trays (2a, 3a) are produced with the aid of the pressure moulding technique from commercially available thermoforming discs of different chemical composition and thickness and are in each case shaped individually in the manner of a dental brace to fit the human maxilla and mandibula.

5. Mandibular protrusion splint according to one of Claims 1 to 4, **characterized in that** the separation between the inside of the bottom surface of the maxillar tray (23) and the inside of the bottom surface of the mandibular tray (22), in the assembled state, is less than 10 mm, preferably from 6 to 9 mm, in particular about 8 mm.

6. Mandibular protrusion splint according to one of Claims 1 to 5, **characterized in that** the separation of the teeth, with the splint in place, in the anterior tooth region is about 8.0 to 8.4 mm.

7. Mandibular protrusion splint according to one of Claims 1 to 6, **characterized in that** the underside of the lower tray (2a) has a scale (12) for reproducible setting of the adjustment screw (10).

8. Ready-to-use set for the production of an appliance for preventing snoring and/or (obstructive) sleep apnoea according to one of Claims 1 to 7, consisting of
(A) a lower part (2) and an upper part (3), comprising a shaping and flexurally rigid tray (2a, 3a) which which are open when applied towards the mandibula and maxilla respectively, wherein the bottom surfaces of each tray exhibit an outside and an inside, and, the outside of the bottom surface of the mandibular tray (2a) has in the centre a screw guide (8) having a thread running parallel to the bottom surface, and the outside of the bottom surfaces of the maxillar tray (3a) has in the centre a guide rail (7) which is open towards the front for accommodation of the screw head of an adjustment screw (10), enabling the two trays (2a, 3a) to be connected to one another in such a way that they are continuously adjustable relative to one another in the longitudinal direction and
(B) two or more adjustment screws (10) of different length.

9. Ready-to-use set for the production of an appliance for preventing snoring and/or (obstructive) sleep apnoea according to one of Claims 1 to 3 and 5 to 7, consisting of
(A) one or more two-part mandibular protrusion splints (1) according to one of Claims 1 to 3 and 5 to 7;
(B) an adapter (14) for the upper part of the two-part mandibular protrusion splint, which is designed in size and shape in such a way that it supports the outside of the bottom surface of the maxillar tray (3a) and having a device mounted on its surface which engages in the guide rail (7), open towards the front, of the maxillar tray (3a); and
(C) optionally use instructions, in which the shaping, adjustment and cleaning of the two-part mandibular protrusion splint are explained.

10. Ready-to-use set for the production or restoration of a mandibular protrusion splint according to one of Claims 2 to 3 and 5 to 7, consisting of
(i) a lower part (2) and an upper part (3), comprising shaping and flexurally rigid trays (2a, 3a) each of which are open when applied towards the mandibula and maxilla respectively, wherein the bottom surfaces of each tray exhibit an outside and an inside, and the outside of the bottom surface of the mandibular tray (2a) has in the centre a screw guide (8) having a thread running parallel to the bottom surface, and the outside of the bottom surfaces of the maxillar tray (3a) has in the centre a guide rail (7) which is open towards the front for accommodation of the screw head of an adjustment screw (10), enabling the two trays (2a, 3a) to be connected to one another in such a way that they are continuously adjustable relative to one another in the longitudinal direction;
(ii) a plastic which is suitable for the production of the thermoplastic filling material (4, 5);
(iii) one or more adjustment screws (10) for connecting the attachment elements; and
(iv) optionally use instructions for the production or restoration of the mandibular protrusion splint;
where (ii) comprises more than the amount of plastic required for the production of the mandibular protrusion splint according to the invention.

11. Ready-to-use set for the production of a mandibular protrusion splint according to one of Claims 1 and 4 to 7, essentially consisting of
(i) a lower part (2) and an upper part (3), comprising a shaping and flexurally rigid tray (2a, 3a) which are open when applied towards the mandibula and maxilla respectively, manufactured by the pressure forming technique after taking of a dental impression, consisting of commercially available polymeric thermoforming discs of different chemical composition and disc thickness, wherein the bottom surfaces of each tray exhibit an outside and an inside;
(ii) a connecting element consisting of a guide rail (7) arranged perpendicular to a baseplate (51) for accommodation of the screw head of an adjustment screw (10) located in the thread of a screw guide (8) arranged perpendicular to a baseplate (52) , where the two baseplates have one or more cut-outs (53) and the shape thereof is matched to the bottom surfaces of the flexurally rigid trays (2a, 3a) enabling them to be affixed thereto and;
(iii) adjustment screws of different length for connecting the individually manufactured trays for maxilla and mandibula, wherein the guide rail (7) which is open towards the front in the assembled state enables the accommodation of the screw head of an adjustment screw located in the thread, enabling the two trays (2a, 3a) to be connected to one another in such a way that they are continuously adjustable relative to one another in the longitudinal direction; and
(iv) optionally use instructions for the production of the mandibular protrusion splint.

## Revendications

1. Orthèse d'avancée mandibulaire en deux parties (1) pour empêcher le ronflement et/ou l'apnée obstructive du sommeil, comprenant une partie inférieure (2) et une partie supérieure (3) composées d'une coque (2a, 3a) respectivement ouverte en cours d'utilisation vers la mâchoire inférieure ou supérieure, imposant une forme et résistant à la flexion, dans laquelle chaque coque présente une surface de fond avec respectivement un côté extérieur et un côté intérieur, **caractérisée en ce que** le côté extérieur de la surface de fond de la coque pour mâchoire inférieure (2a) présente au milieu un guidage de vis (8) avec un filetage s'étendant en parallèle à la surface de fond, et le côté extérieur de la surface de fond de la coque pour mâchoire supérieure (3a) présente au milieu un rail de guidage (7) ouvert vers l'avant pour recevoir la tête de vis d'une vis de réglage (10) se trouvant dans le filetage, ce qui permet de relier les deux coques (2a, 3a) entre elles de telle sorte qu'elles sont réglables l'une par rapport à l'autre en continu dans la direction longitudinale.

2. Orthèse d'avancée mandibulaire selon la revendication 1, **caractérisée en ce que** les coques (2a, 3a) résistant à la flexion contiennent une matière de remplissage thermoplastique (4, 5) qui peut être modelée respectivement à la manière d'un appareil dentaire selon la mâchoire humaine supérieure et inférieure.

3. Orthèse d'avancée mandibulaire selon la revendication 1 ou 2, **caractérisée en ce que** dans la matière de remplissage (4) de la coque pour mâchoire supérieure, un creux d'orientation (13) est formé pour marquer l'emplacement d'occlusion, en particulier pour les incisives.

4. Orthèse d'avancée mandibulaire selon la revendication 1, **caractérisée en ce que** les coques (2a, 3a) résistant à la flexion sont fabriquées à l'aide de la technique de moulage par compression à partir de plaques d'emboutissage profond du commerce de compositions chimiques et épaisseurs différentes et sont respectivement modelées à la manière d'un appareil dentaire individuellement selon la mâchoire humaine supérieure et inférieure.

5. Orthèse d'avancée mandibulaire selon l'une quelconque des revendications 1 à 4, **caractérisée en ce que** l'écartement entre le côté intérieur de la surface de fond de la coque pour mâchoire supérieure (23) et le côté intérieur de la surface de fond de la coque pour mâchoire inférieure (22) mesure à l'état assemblé moins de 10 mm, de préférence de 6 à 9 mm, en particulier environ 8 mm.

6. Orthèse d'avancée mandibulaire selon l'une quelconque des revendications 1 à 5, **caractérisée en ce que** l'écartement des dents lorsque le rail est appliqué se situe au niveau des incisives à environ 8,0 à 8,4 mm.

7. Orthèse d'avancée mandibulaire selon l'une quelconque des revendications 1 à 6, **caractérisée en ce que** la surface de fond de la coque pour mâchoire inférieure (2a) présente une échelle (12) pour le réglage reproductible de la vis de réglage (10).

8. Kit prêt à l'emploi pour la fabrication d'une orthèse d'avancée mandibulaire pour empêcher le ronflement et/ou l'apnée (obstructive) du sommeil selon l'une quelconque des revendications 1 à 7, comprenant
(A) une partie inférieure (2) et une partie supérieure (3), composées d'une coque (2a, 3a) respectivement ouverte en cours d'utilisation vers la mâchoire inférieure ou supérieure, imposant une forme et résistant à la flexion, dans laquelle les surfaces de fond de ces coques présentent respectivement un côté extérieur et un côté intérieur, et le côté extérieur de la surface de fond de la coque pour mâchoire inférieure (2a) présente au centre un guidage de vis (8) avec un filetage s'étendant en parallèle à la surface de fond, et le côté extérieur de la surface de fond de la coque pour mâchoire supérieure (3a) présente au centre un rail de guidage (7) ouvert vers l'avant pour recevoir la tête de vis d'une vis de réglage (10) pouvant être vissée dans le filetage, ce qui permet de relier les deux coques (2a, 3a) entre elles de telle sorte qu'elles sont réglables l'une par rapport à l'autre en continu dans la direction longitudinale ;
(B) deux ou plusieurs vis de réglages (10) de longueurs différentes.

9. Kit prêt à l'emploi pour la fabrication d'une orthèse d'avancée mandibulaire pour empêcher le ronflement et/ou l'apnée (obstructive) du sommeil selon l'une quelconque des revendications 1 à 3 ainsi que 5 à 7, comprenant
(A) une ou plusieurs orthèses d'avancée mandibulaire en deux parties (1) selon l'une quelconque des revendications 1 à 3 ainsi que 5 à 7 ;
(B) un dispositif d'adaptation (14) pour la partie supérieure des orthèses d'avancée mandibulaire en deux parties, qui est configuré en dimension et forme de telle sorte qu'il soutient le côté extérieur de la surface de fond de la coque pour mâchoire supérieure (3a), et s'engage avec un dispositif monté sur sa surface dans le rail de guidage (7), ouvert vers l'avant, de la coque pour mâchoire supérieure (3a) ; et
(C) éventuellement une notice d'emploi, dans laquelle le moulage, l'ajustage et le nettoyage des orthèses d'avancée mandibulaire en deux parties sont expliqués.

10. Kit prêt à l'emploi pour la fabrication ou la réparation d'une orthèse d'avancée mandibulaire selon l'une quelconque des revendications 2 à 3 et 5 à 7, comprenant
(i) une partie inférieure (2) et une partie supérieure (3), composées d'une coque (2a, 3a) respectivement ouverte en cours d'utilisation vers la mâchoire inférieure ou supérieure, imposant une forme et résistant à la flexion, dans laquelle les surfaces de fond de ces coques présentent respectivement un côté extérieur et un côté intérieur, et le côté extérieur de la surface de fond de la coque pour mâchoire inférieure (2a) présente au milieu un guidage de vis (8) avec un filetage s'étendant en parallèle à la surface de fond, et le côté extérieur de la surface de fond de la coque pour mâchoire supérieure (3a) présente au milieu un rail de guidage (7) ouvert vers l'avant pour recevoir la tête de vis d'une vis de réglage (10) pouvant être vissée dans le filetage, ce qui permet de relier les deux coques (2a, 3a) entre elles de telle sorte qu'elles sont réglables l'une par rapport à l'autre en continu dans la direction longitudinale ;
(ii) une matière plastique appropriée pour la fabrication de la matière de remplissage thermoplastique (4, 5) ;
(iii) une ou plusieurs vis de réglage (10) pour relier les deux coques (2a, 3a) ; et
(iv) éventuellement une notice d'emploi pour la fabrication ou la réparation de l'orthèse d'avancée mandibulaire ;
dans lequel (ii) comprend plus que la quantité de matière plastique nécessaire à la fabrication de l'orthèse d'avancée de mâchoire inférieure selon l'invention.

11. Kit prêt à l'emploi pour la fabrication d'une orthèse d'avancée mandibulaire selon l'une quelconque des revendications 1 et 4 à 7, comprenant substantiellement
(i) une partie inférieure (2) et une partie supérieure (3), composées d'une coque (2a, 3a) respectivement ouverte en cours d'utilisation vers la mâchoire inférieure ou supérieure, imposant une forme et résistant à la flexion, qui ont été fabriquées respectivement, après réalisation d'une empreinte dentaire, à partir de plaques d'emboutissage profond polymères du commerce de composition chimique et d'épaisseur de plaque différentes par une technique de moulage par compression, dans lequel les surfaces de fond de ces coques présentent respectivement un côté extérieur et un côté intérieur ;
(ii) un élément de liaison, composé d'un rail de guidage (7) disposé perpendiculairement à une plaque de fond (51) pour recevoir la tête de vis d'une vis de réglage (10) pouvant être vissée dans le filetage (10), d'un guidage de vis (8) disposé perpendiculairement à une plaque de fond (52), dans lequel les deux plaques de fond présentent un ou plusieurs évidements (53) et leur forme est adaptée aux côtés extérieurs des surfaces de fond du côté extérieur respectif des coques (2a, 3a) résistant à la flexion de telle sorte qu'ils peuvent y être montés respectivement au milieu ; et
(iii) des vis de réglage de longueur différente pour relier les coques pour mâchoire supérieure et inférieure, fabriquées individuellement, dans lequel le rail de guidage (7) ouvert vers l'avant à l'état assemblé permet de recevoir la tête de vis d'une vis de réglage se trouvant dans le filetage, et ainsi celles-ci peuvent être reliées entre elles de telle sorte que les deux coques (2a, 3a) sont réglables l'une par rapport à l'autre en continu dans la direction longitudinale ; et
(iv) éventuellement une notice d'emploi pour la fabrication de l'orthèse d'avancée mandibulaire.
